# EUROPÄISCHE PATENTSCHRIFT

(11) **EP 1 033 361 B1**
(45) Veröffentlichungstag und Bekanntmachung des Hinweises auf die Patenterteilung: **04.12.2002**
(21) Anmeldenummer: 00102504.8
(22) Anmeldetag: 05.02.2000
(51) Int. Cl.: C07C 209/36, C07C 211/50

(54) **Verfahren zur Herstellung von Aminen**
Process for the preparation of amines
Procédé pour préparer d'amines

(30) Priorität: 03.03.1999 DE 19909168
(43) Veröffentlichungstag der Anmeldung: 06.09.2000
(73) Patentinhaber: BASF AKTIENGESELLSCHAFT, 67056 Ludwigshafen (DE)
(72) Erfinder: Sander, Michael, Dr., 01945 Ruhland (DE); Peisker, Dietmar, 01968 Senftenberg (DE); Werner, Klaus, 01987 Schwarzheide (DE); Braunsberg, Holger, 01968 Senftenberg (DE); Georgi, Gunter, Dr., 01979 Lauchhammer (DE); Penzel, Ulrich, Dr., 01945 Tettau (DE)

(56) Entgegenhaltungen:
- EP-A- 0 335 222
- GB-A- 1 033 651

## Beschreibung

Die Erfindung betrifft ein Verfahren zur Herstellung von Aminen durch Hydrierung der entsprechenden Nitroverbindungen.

### Stand der Technik

Die Herstellung von Aminen, insbesondere von aromatischen Mono-, Di- und/oder Polyaminen durch katalytische Hydrierung der entsprechenden Mono-, Di- und/oder Polynitroverbindungen ist seit langem bekannt und vielfach in der Literatur beschrieben.

Bei der in der Technik üblichen Herstellung von Mono-, Di- und/oder Polyaminen durch Umsetzung der entsprechenden Nitroverbindungen mit Wasserstoff wird eine beträchtliche Wärmemenge frei. Zumeist wird die Hydrierung daher in der Technik bei möglichst niedrigen Temperaturen unter Einsatz von Hydrierkatalysatoren in der Flüssigphase durchgeführt. Dabei wird die zu reduzierende Verbindung in einem Lösungsmittel mit dem Katalysator vermischt und diskontinuierlich in einem Autoklaven oder kontinuierlich in einem Schlaufenreaktor, einer Blasensäule oder einer Reaktorkaskade reduziert. Diese katalytischen Hydrierverfahren arbeiten üblicherweise bei Temperaturen von 353 bis 500 K und Drücken von 5·10⁵ bis 5·10⁶ Pa unter Zusatz von Lösungsmitteln, wie Wasser, Alkoholen, Kohlenwasserstoffen oder bereits hydrierten Produkten. Bei diesen bisher bekannten Verfahren gibt es eine Reihe von Nachteilen, wie z.B. die Notwendigkeit des Austragens und besonders des Ausschleusens desaktivierter Katalysatoranteile, was zu Katalysatorverlusten führt. Ferner stellen die häufig auftretenden Nebenreaktionen, die zur Bildung störender Substanzen, wie z.B. teerartiger Bestandteile, und damit zu Ausbeuteminderungen führen, ein Problem vieler bislang verwendeter Verfahren dar. Als Hydrierkatalysatoren werden, wie beispielsweise in EP-A-0 124 010 beschrieben, vorzugsweise Metalle der VIII. Nebengruppe des Periodensystems, insbesondere Raney-Eisen, Raney-Kobalt und Raney-Nickel, eingesetzt.

Um diese Nachteile zu verringern, ist es bekannt, den Katalysator in einem Festbett anzuordnen. So beschreibt DE-OS 2 135 154 die Hydrierung einer Nitroverbindung allein oder in Anwesenheit eines Verdünnungsmittel in flüssigem Zustand in einem Röhrenreaktor in Anwesenheit eines Palladium-Katalysators auf Spinell in einem Festbett. Bei der Verwendung dieses Palladium-Katalysators auf Spinell ist die Katalysatorherstellung sehr aufwendig, und eine gezielte Fixierung auf dem Träger nur teilweise möglich. Ferner führt diese Festbetthydrierung zu geringen Hydrierausbeuten und zur Bildung von hochsiedenden Nebenprodukten. Beispielhaft erwähnt seien in diesem Zusammenhang hydrogenolytische Spaltungen, Kernhydrierungen oder die Bildung von hochmolekularen, teerartigen Substanzen. Es können infolge des stark exothermen Reaktionsverlaufs der Nitrogruppenumsetzung und der hohen Reaktionsgeschwindigkeit bei höheren Temperaturen auch explosionsartige Nebenreaktionen auftreten.

Um diese unerwünschten Nebenreaktionen soweit wie möglich auszuschließen, wurde daher im allgemeinen die großtechnische Hydrierung von Nitroverbindungen bei relativ niedrigen Temperaturen durchgeführt.

Die günstigste bekannte Verfahrensvariante der Hydrierung von Nitroverbindungen ist das kontinuierliche Sumpfphasenverfahren mit einem Kreislauf des Reaktionsgemisches zwischen einem Reaktor mit intensiver Rührung und einem Dekantiergefäß zur Rückführung des Katalysators in den Reaktor. Die dabei eingesetzten Katalysatoren werden unter der mechanischen Belastung im Reaktor jedoch relativ schnell zu Korngrößen zerkleinert, die im Schwerkraftabscheider nicht mehr sedimentiert werden können und mit dem Hydrierprodukt aus dem oberen Teil des Dekantiergefäßes zu einem hohen Anteil in die Aufarbeitung abgenommen werden müssen. Die bekannten Verfahren (BE-A-0 846 341) arbeiten außerdem bei relativ hohen Katalysatorkonzentrationen in der Reaktionslösung. Die hohen Katalysatorkonzentrationen im Reaktionsgemisch während der Hydrierung sind erforderlich, da eine Steuergröße zur Regulierung der Nitrogruppenkonzentration im Reaktor fehlt. Die Reaktoren werden deshalb unter solchen Bedingungen betrieben, die gewährleisten, daß die gesamte in das Reaktionssystem eingespeiste Menge an Nitroverbindung sofort hydriert wird, so daß die Nitrogruppenkonzentration im Reaktionsgemisch also etwa bei Null liegt.

Als Katalysatoren finden bei den beschriebenen Verfahren verschiedene Katalysatorsysteme Anwendung; am häufigsten werden Ni-haltige Katalysatoren eingesetzt. Die Hydrierung von 2,4- und 2,6-Dinitrotoluol in Gegenwart der Katalysatoren Pd/C, Raney-Nickel, Raney-Kobalt bzw. Platinschwarz ist beispielsweise in JP-A-5 513 333 offenbart. Ein Ni-Kieselgur-Trägerkatalysator wird für die Hydrierung von Di-Nitrobenzophenon zum Diamin in EP-A-98 681 beschrieben.

In der DE 3 537 247 A wird die Hydrierung von Dinitroverbindungen zu den Diaminen in Gegenwart von modifizierten Raney-Nickel-Katalysatoren beschrieben. Das bevorzugte Katalysatorsystem enthält neben Nickel, Eisen, Chrom, Kupfer, Molybdän, Wolfram, Vanadium, Titan, Niob, Rhenium, Ruthenium, Zirkon und/oder Hafnium.

In der DD 152 065 wird der Einsatz eines Ni-SiO₂-Katalysators mit spezieller Partikelgrößenverteilung für die Hydrierung von Nitroaromaten gelehrt.

EP 0 335 222 lehrt die Anwendung von Ni-Al₂O₃/ZrO₂-Trägerkatalysatoren für die Hydrierung von Nitrilen, Aromaten, Nitroverbindungen und Olefinen. Beansprucht wird insbesondere die gleichzeitige Fällung von Ni, Zr und Al auf Trägern, wobei besonderes Augenmerk auf eine homogene Ausfällung der Komponenten gelegt wird.

Ni-Al₂O₃-ZrO₂ Katalysatoren werden in der SU-PS 283 185 beschrieben. Sie werden durch Fällen von Ni und Al₂O₃ auf ZrO₂ hergestellt.

Gemäß der Lehre der US-PS 2 564 331 wird ein Ni-ZrO₂ Katalysator durch Fällen eines Nickel- und Zirkoncarbonatgemisches mit anschließendem Waschen, Trocknen und Reduzieren bei 250 bis 350°C hergestellt. Der Katalysator enthält maximal 10% ZrO₂.

In der DE-AS 1 257 753 wird ebenfalls die Fällung der unlöslichen Carbonate beschrieben. Der Fällvorgang wird durch Verdampfen von CO₂ und NH₃ aus einer Mischsalzlösung von Ammoniumzirkonylcarbonat und Nickelamincarbonat ausgelöst.

EP 0 672 452 offenbart Katalysatoren zur Hydrierung organischer Verbindungen, die im wesentlichen 65 bis 80% Ni, berechnet als NiO, 10 bis 25% Silicium, berechnet als SiO₂, 2 bis 10% Zirkon, berechnet als ZrO₂, und 0-10% Aluminium berechnet als Al₂O₃, enthalten, wobei die Summe aus dem Gehalt an SiO₂ und Al₂O₃ mindestens 15% beträgt.

Nachteilig an allen bisher bekannten Ni-haltigen Katalysatorsystemen ist die unzureichende mechanische und/oder chemische Stabilität des eingesetzten Trägermaterials. So stellen Katalysatorabrieb, hoher Feinkornanteil, schlechte Sedimentationseigenschaften, Katalysatoraustrag, Auslaugung des Trägermaterials und/oder rasche Desaktivierung des Katalysators die typischen Probleme aller bislang verwendeten Verfahren zur Hydrierung von Nitroverbindungen in Suspensionsfahrweise dar.

Die Leistungsgrenze des zur Hydrierung eingesetzten Katalysators ist während der Reaktion nur ungenau zu erkennen. Bei Erreichen der Leistungsgrenze des Katalysators erfolgt ein außerordentlich schneller Anstieg der Nitrogruppenkonzentration. Die steigenden Konzentrationen an Nitroverbindung wirken wiederum vergiftend auf die im Reaktionssystem enthaltenen Katalysatormengen. Dadurch sinkt die Reaktionsgeschwindigkeit weiter und die Konzentration an Nitroverbindungen nimmt dementsprechend bei gleichbleibender Einspeisung von Nitroverbindungen noch schneller zu. Die bekannten Methoden zur Kontrolle und Regelung der Nitrogruppenkonzentration arbeiten zu träge, um den schnellen Anstieg der Nitrogruppenkonzentration in diesen Fällen zu vermeiden. Auch wenn eine ausreichend niedrige Konzentration an Nitroverbindungen im Reaktionsgemisch gemessen wurde, so sagt dieser Meßwert nichts darüber aus, ob bereits die Leistungsgrenze des Katalysators erreicht bzw. überschritten wurde. Liegen erst einmal hohe Nitrogruppenkonzentrationen in der Reaktionsmischung vor, so besteht die Gefahr, daß bei weiterer Zugabe von Katalysator in das Reaktionssystem eine starke Aufheizung des Reaktionsgemisches infolge der hohen Reaktionsgeschwindigkeit auftreten kann. Die dabei in relativ kurzer Zeit freigesetzte Reaktionswärme ist von dem im Reaktor installierten Wärmetauschersystem dann nicht mehr abzuleiten. Es tritt ein sprungartiger Anstieg des Druckes im Reaktor auf, der zur Zerstörung der Anlage führen kann. Die alleinige Kontrolle der Nitrogruppenkonzentration im Reaktionsgemisch ergibt deshalb keine ausreichende Sicherheit.

Noch kritischer stellen sich diese Sicherheitsprobleme dar, wenn man berücksichtigt, daß die katalytische Aktivität der eingesetzten Katalysatoren durch geringe Veränderungen bei ihrer Herstellung oder ihrer Aktivierung selbst innerhalb einer Herstellungscharge stark schwanken kann.

Bei den bekannten Verfahren zur Hydrierung von Nitroverbindungen versucht man, die Sicherheit des Reaktionssystems durch einen beträchtlichen Überschuß an Katalysator über den reaktionskinetisch erforderlichen Anteil hinaus zu erreichen. Bei den bekannten Verfahren wird zumeist mit Katalysatorkonzentrationen im Bereich zwischen 5 bis 15 Gew.-%, bezogen auf die Reaktionsmischung, gearbeitet. Durch diese hohe Katalysatorkonzentration gelingt es mit hoher Wahrscheinlichkeit, die gesamte kontinuierlich zudosierte Menge an Nitroverbindung vollständig zu hydrieren. Eine Anreicherung an Nitroverbindungen im Reaktionsgemisch, die zu einer starken Wärmeentwicklung und damit zu einem Durchgehen der Reaktion im Reaktionsbehälter führen kann, wird damit weitgehend vermieden. Wesentlicher Nachteil dieser Verfahrensweise ist jedoch ein hoher Katalysatorverbrauch. Die hohen Katalysatorkonzentrationen im Reaktionsgemisch haben außerdem zur Folge, daß es an strömungsungünstigen Reaktorteilen und an Verengungen im Reaktionssystem zu Ablagerungen und Verfestigungen von Katalysator kommt. Diese Ablagerungen führen zu Verstopfungen im Reaktor, die das Abstellen der gesamten Hydrierung verursachen können. Schließlich bewirken die hohen Katalysatorkonzentrationen im Reaktionsgemisch auch einen erhöhten Austrag an Katalysator aus dem Reaktor. Die mit dem hydrierten Produkt ausgetragenen Katalysatoranteile sind hochaktiv, können jedoch nicht wieder in das Reaktionssystem zurückgeführt werden. Sie verursachen andererseits erhebliche Schwierigkeiten bei der Weiterverarbeitung der Hydrierprodukte, zum Beispiel in den Destillationskolonnen. In den Anlagen zur Hydrierung der Nitroverbindungen mit Kreislauffahrweise des Hydrierprodukts zwischen einem Hydrierreaktor und einem Sedimentationsbehälter zur Rückführung des sedimentierten Katalysators treten bei Einsatz von SiO₂-und/oder Al₂O₃- geträgerten Katalysatoren und insbesondere bei höheren Reaktionstemperaturen ohne Zusatz von alkoholischen Lösungsmitteln, Kohlenwasserstoffen etc. Verstopfungen in der Rückführleitung zwischen Reaktor und Schwerkraftabscheider auf, da der amphotere Katalysatorträger durch das basische Reaktionsmedium ausgelaugt wird, was zu Katalysatoragglomerationen und -verklebungen führt. Dies bedeutet einen zusätzlichen Verlust an hydrieraktiver Oberfläche und damit eine weitere Verringerung der Standzeit des Katalysators. Ebenso ist die Bildung von Ni-haltigen Silikaten und/oder -aluminaten zu beobachten, welche in erster Linie die Wärmetauscher der Produktionsanlage belegen.

Ein weiterer Nachteil der bekannten Verfahren mit hohen Katalysatorkonzentrationen ist die erhöhte Bildung von Nebenprodukten, was durch den Zusatz von alkoholischen Lösungsmitteln noch begünstigt wird. Dadurch wird die Ausbeute an dem erwünschten Hydrierprodukt stark vermindert. Hydrierprodukte mit hohem Anteil an Nebenprodukten verursachen außerdem enorme Mehraufwendungen zur Reinigung der gewünschten Endprodukte. Weiterhin entstehen zusätzliche Verluste an Amin, da in den abgetrennten Nebenprodukten noch erhebliche Mengen der gewünschten Amine enthalten sind, wenn der Aufwand zur Reinigung nicht zu hoch liegen soll. Der Verzicht auf zusätzliche Lösungsmittel ist daher wünschenswert.

Der vorliegenden Erfindung liegt die Aufgabe zugrunde, ein für den technischen Einsatz geeignetes, verbessertes Verfahren zur störungsfreien Herstellung von aromatischen und/oder aliphatischen Aminen mit sehr guten Verarbeitungseigenschaften durch katalytische Hydrierung der den Aminen zugrunde liegenden Nitroverbindungen bei üblichen Verfahrensparametern und mit üblichen Reaktoren unter Einsatz von Hydrierkatalysatoren in der Sumpfphase, in Anwesenheit oder Abwesenheit eines Lösungsmittels, mit niedrigem Katalysatorverbrauch unter weitgehender Vermeidung von Nebenreaktionen und unter Einstellung einer optimalen Katalysatorbilanz sowie einer optimalen Separierung inaktiver Katalysatoranteile zu entwickeln.

Diese Aufgabe konnte durch das erfindungsgemäße Verfahren gelöst werden, wobei ein spezieller Hydrierkatalysator verwendet wird.

Gegenstand der Erfindung ist ein Verfahren zur Herstellung von Aminen, insbesondere aromatischen Aminen, wobei mindestens eine Verbindung, die mindestens eine Nitrogruppe aufweist, in Gegenwart eines Trägerkatalysators, der als katalytisch aktives Metall Nickel aufweist, hydriert wird, dadurch gekennzeichnet, daß der reduzierte und stabilisierte Trägerkatalysator Nickel-Kristallite mit bimodaler Nickel-Kristallitgrößenverteilung mit Maxima bei 30 - 80 Angström und 81 - 150 Angström auf einem Träger aus ZrO₂, ZrO₂₋HfO₂ und/oder SiO₂-ZrO₂ und/oder SiO₂-ZrO₂-HfO₂ enthält und im reduzierten und passivierten Zustand einen Nickelgehalt von 60-80 Masseprozent, einen SiO₂-Gehalt von 0 - 20 Masseprozent, einen ZrO₂-Gehalt von 0 - 40 Masseprozent, einen HfO₂-Gehalt von 0 - 4 Masseprozent aufweist, wobei der Gehalt mindestens einer der Verbindungen, aus denen der Träger aufgebaut ist, ungleich 0 Masseprozent beträgt, und nach einer einstündigen Nachreduktion bei 100°C einen Reduktionsgrad von mindestens 70 % besitzt.

Unter einer bimodalen Korngrößenverteilung wird eine Korngrößenverteilung verstanden, deren Verteilungskurve zwei Maxima aufweist.

Unter dem reduzierten und passivierten Zustand des Katalysators wird verstanden, daß der Katalysacor nach der Herstellung aktiviert wurde, danach jedoch, da er in diesem Zustand zumeist nicht lagerfähig ist, die aktiven Zentren, beispielsweise durch Überleiten von Sauerstoff oder Kohlendioxid, passiviert werden. Der erfindungsgemäß verwendete Katalysator wird Vorzugsweise in einer Menge von 0,1 bis 5, vorzugsweise 0,2 bis 2 Gew.-%, bezogen auf die Reaktionsmischung, eingesetzt.

Unter Reaktionsmischung wird hierbei die Gesamtmenge der im Reaktor befindlichen Verbindungen verstanden, unter anderem Ausgangsund Endprodukt, Lösungsmittel und Wasser.

Das erfindungsgemäße Verfahren kann kontinuierlich oder diskontinuierlich unter Verwendung üblicher Reaktoren bei üblichen Verfahrensparametern wie Druck und Temperatur, vorzugsweise niedrigen Temperaturen, durchgeführt werden.

Vorzugsweise wird die erfindungsgemäße Hydrierung bei Drücken im Bereich von 10 bis ungefähr 40 bar, weiter bevorzugt bei 20 bis 25 bar, durchgeführt.

Bevorzugt wird die erfindungsgemäße Hydrierung im Bereich von 80 bis 200 °C, besonders bevorzugt im Bereich von 90 bis 160 °C und insbesondere im Bereich 100 bis 140 °C durchgeführt.

Zumeist wird die Hydrierung in Form einer kontinuierlichen Sumpfphasenhydrierung in üblichen und geeigneten Reaktoren durchgeführt. Als Reaktoren kommen beispielsweise Rührkessel oder Schlaufenreaktoren, wie zum Beispiel Strahlschlaufenreaktoren, sogenannte Loop-Venturi-Reaktoren, zum Einsatz.

Als Hydriergase können beliebige Gase verwendet werden, die freien Wasserstoff enthalten und keine schädlichen Mengen an Katalysatorgiften wie beispielsweise CO aufweisen. So können z.B. Reformerabgase verwendet werden. Vorzugsweise wird jedoch reiner Wasserstoff als Hydriergas verwendet.

Die bei der Hydrierung gebildeten Amine werden dem Hydriervorgang kontinuierlich oder diskontinuierlich entzogen und einer Aufarbeitung, beispielsweise einer destillativen Nachbehandlung, unterworfen.

Vorzugsweise werden nach dem erfindungsgemäßen Verfahren aromatische Nitroverbindungen mit einer oder mehreren Nitrogruppen und 1 bis 18, vorzugsweise 6 bis 18 Kohlenstoffatomen im Molekül, beispielsweise Nitrobenzole, wie z.B. o-, m-, p-Nitrobenzol, 1,3-Dinitrobenzol, Nitrotoluole, wie z.B. 2,4-, 2,6-Dinitrotoluol, 2,4,6-Trinitrotoluol, Nitroxylole, wie z.B. 1,2-Dimethyl-3-, 1,2 Dimethyl-4-, 1,4-Dimethyl-2-, 1,3-Di-methyl-2-, 2,4-Dimethyl-1- und 1,3-Dimethyl-5-nitrobenzol, Nitronaphthaline, wie z.B. 1-, 2-Nitronaphthalin, 1,5 und 1,8-Dinitronaphthalin, Chlornitrobenzole, wie z.B. 2-Chlor-1,3-, 1-Chlor-2,4-dinitrobenzol, o-, m-, p-Chlornitrobenzol, 1,2-Dichlor-4-, 1,4-Dichlor-2-, 2,4-Dichlor-1-und 1,2-Dichlor-3-nitrobenzol, Chlornitrotoluole, wie z.B. 4-Chlor-2, 4-Chlor-3-, 2-Chlor-4- und 2-Chlor-6-nitrotoluol, Nitroaniline, wie z.B. o-, m-, p- Nitroanilin; Nitroalkohole, wie z.B. Tris(hydroxymethyl)-nitromethan, 2-Nitro-2-methyl-, 2-Nitro-2-ethyl-1,3-propandiol, 2-Nitro-1-butanol und 2-Nitro-2-methyl-1-propanol sowie beliebige Gemische aus zwei oder mehreren der genannten Nitroverbindungen eingesetzt.

Bevorzugt werden nach dem erfindungsgemäßen Verfahren aromatische Nitroverbindungen, vorzugsweise Mononitrobenzol, Methylnitrobenzol oder Dimethyldinitrobenzol, und insbesondere 2,4-Dinitrotoluol oder dessen technische Gemische mit 2,6-Dinitrotoluol, wobei diese Gemische vorzugsweise bis zu 35 Gewichtsprozent, bezogen auf das Gesamtgemisch, an 2,6-Dinitrotoluol mit Anteilen von 1 bis 4 Prozent an vicinalem DNT und 0,5 bis 1,5 % an 2,5- und 3,5-Dinitrotoluol aufweisen, zu den entsprechenden Aminen hydriert.

Im erfindungsgemäßen Verfahren kann die aromatische und/oder aliphatische Nitroverbindung, vorzugsweise Di- und/oder Polynitroverbindung in reiner Form, als Mischung mit dem entsprechenden Di- und/oder Polyamin, als Mischung mit dem entsprechenden Di- und/oder Polyamin und Wasser, als Mischung mit dem entsprechenden Di- und/oder Polyamin, Wasser und einem alkoholischen Lösungsmittel oder als Mischung mit dem entsprechenden Di- und/oder Polyamin, Wasser, einem alkoholischen Lösungsmittel und einem katalysatorreaktivierenden Zusatz eingesetzt werden, wobei jeweils auch Gemische aus zwei oder mehr der oben genannten Nitroverbindungen, der entsprechenden Aminverbindungen, dem alkoholischen Lösungsmittel und dem katalysatorreaktivierenden Zusatz eingesetzt werden können.

Sofern ein oben beschriebenes Gemisch eingesetzt wird, liegt das Verhältnis von Aminverbindung zu Wasser vorzugsweise im Bereich von 10:1 bis 1:10, besonders bevorzugt im Bereich von 2:1 bis 1:2 und das Verhältnis des Amin/Wasser-Gemischs zu mindestens einem alkoholischen Lösungsmittel vorzugsweise bei 500:1 bis 1:1, besonders bevorzugt bei 50:1 bis 5:1.

Wie sich aus dem oben gesagten ergibt, kann beim erfindungsgemäßen Verfahren die Hydrierung unter Abwesenheit oder in Gegenwart eines alkoholischen Lösungsmittels und eines katalysatorreaktivierenden Zusatzes durchgeführt werden.

Sofern ein alkoholisches Lösungsmittel und ein katalysatorreaktivierender Zusatz verwendet werden, können selbstverständlich auch Gemische aus zwei oder mehr davon zugesetzt werden.

Als alkoholische Lösungsmittel werden niedere aliphatische Alkohole mit 1 bis 6 C-Atomen, vorzugsweise Methanol, Ethanol oder Propanol einzeln oder ein Gemisch aus zwei oder mehr davon verwendet.

Als katalysatorreaktivierende Zusätze werden vorzugsweise aprotische Lösungsmittel, insbesondere Aceton, DMF, Dioxan oder THF oder ein Gemisch aus zwei oder mehr davon eingesetzt.

Die Menge der eingesetzten alkoholischen Lösungsmittel und der katalysatorreaktivierende Zusätze ist im Rahmen des erfindungsgemäßen Verfahrens nicht in besonderer Weise beschränkt und kann je nach Bedarf frei gewählt werden.

Überraschenderweise ist es jedoch auch möglich, die Hydrierung aromatischer Nitroverbindungen nach dem erfindungsgemäßen Verfahren ohne die Verwendung von Lösungsmitteln durchzuführen. Bei dieser Verfahrensweise vereinfacht sich die Aufarbeitung des Reaktionsgemisches nach der Hydrierung, außerdem werden Nebenreaktionen mit dem Lösungsmittel völlig unterbunden.

Das erfindungsgemäße Verfahren wird, wie oben ausgeführt, in Gegenwart eines Trägerkatalysators, der als Aktivkomponente Nickel alleine oder zusammen mit mindestens einem Metall der I., V., VI. und/oder VIII. Nebengruppe aufweist, durchgeführt.

Die erfindungsgemäß verwendeten Katalysatoren können technisch hergestellt werden durch Aufbringen von Nickel und ggf. mindestens einem der oben genannten zusätzlichen Metalle auf einen geeigneten Träger.

Vorzugsweise werden als Metalle der I., V., VI. und/oder VIII. Nebengruppe des Periodensystems Palladium, Platin, Rhodium, Eisen, Kobalt, Chrom, Vanadium, Kupfer, Silber oder ein Gemisch aus zwei oder mehr davon verwendet.

Als Trägermaterialien werden vorzugsweise Siliciumdioxid, Siliciumcarbid, Kieselgur, Aluminiumoxid, Magnesiumoxid, Titandioxid, Zirkoniumdioxid und/oder Hafniumdioxid oder ein Gemisch aus zwei oder mehr davon, besonders bevorzugt Zirkoniumdioxid, ZrO₂₋HfO₂ und/oder SiO₂₋ZrO₂ und/oder SiO₂-ZrO₂₋HfO₂, verwendet.

Die verwendeten Träger sind vorzugsweise mesoporös und besitzen einen mittleren Porendurchmesser von 35 - 50 nm und eine spezifische Oberfläche von 50 - 250 m²/g. Die Oberfläche des Trägers wird bestimmt nach dem BET-Verfahren durch N₂-Adsorption, insbesondere nach DIN 66131. Die Bestimmung des mittleren Porendurchmessers und der Porengrößenverteilung erfolgt durch Hg-Porosimetrie, insbesondere nach DIN 66133.

Das Aufbringen von Nickel und gegebenenfalls des mindestens einen weiteren Metalls kann durch die üblichen geeigneten Verfahren erreicht werden. Die mit dem Metall bzw. Metallsalz beschichteten oder getränkten Träger werden anschließend nach bekannten Verfahren getrocknet und calciniert. Darauffolgend werden die beschichteten Träger durch Behandlung derselben in einem Gasstrom, der freien Wasserstoff enthält, aktiviert. Diese Aktivierung findet zumeist bei Temperaturen zwischen 30 und 600 °C, vorzugsweise zwischen 80 und 150 °C und besonders bevorzugt bei 100 °C statt. Der Gasstrom besteht vorzugsweise aus 50 bis 100 Vol.-% Wasserstoff und 0 bis 50 Vol.-% Stickstoff. Der für den erfindungsgemäßen Einsatz hergestellte Katalysator weist nach einstündiger Reduktion bei 100 °C einen Reduktionsgrad von mindestens 70 % auf.

Die so erhaltenen Trägerkatalysatoren besitzen im allgemeinen eine Nickel-Metalloberfläche von ungefähr 10 bis ungefähr 150 m²/g, vorzugsweise ungefähr 20 bis ungefähr 60 m²/g und einen Nickelgehalt von im allgemeinen ungefähr 50 bis ungefähr 80 Gew.-%, vorzugsweise ungefähr 60 bis ungefähr 75 Gew.-%.

Da diese aktivierten Katalysatoren sehr reaktiv und bei Zutritt von Luftsauerstoff bzw. Feuchtigkeit oft selbstentzündlich sind, ist es üblich, diese zu passivieren. Dies kann beispielsweise durch Überleiten von Sauerstoff oder Kohlendioxid erfolgen.

Der erfindungsgemäß verwendete Trägerkatalysator weist, wie bereits ausgeführt, Nickel in einer bimodalen Kristallitgrößenverteilung im Bereich von ungefähr 1 bis ungefähr 200 Angström mit Maxima bei ungefähr 30 bis ungefähr 80 Angström und bei ungefähr 81 bis ungefähr 150 Angström auf, wobei der Anteil des feindispersen Nickels (Maxima der Nickel-Kristallitgrößenverteilung bei 30 bis 80 Angström) ≥ 40 % beträgt.

Das erfindungsgemäße Verfahren hat den Vorteil, daß durch den Einsatz der beschriebenen Katalysatoren die Hydrierung in einem weiten Temperaturbereich unter maximaler Nutzung der hohen katalytischen Aktivität des eingesetzten Katalysators störungsfrei und mit hoher Raum-Zeit-Ausbeute gefahren werden kann. Das erfindungsgemäße Verfahren ermöglicht es, die Nitroaromatenhydrierung mit wesentlich geringeren Katalysatorkonzentrationen im Hydrierbad durchzuführen als die bekannten Verfahren. Dadurch können erhebliche Einsparungen an Katalysator im Vergleich zu den bekannten Verfahren erreicht werden. Außerdem wird durch die geringe Katalysatorkonzentration und den geringen Katalysatoreinsatz bei dem erfindungsgemäßen Verfahren eine minimale Nebenproduktbildung, insbesondere von teerartigen Produkten, erreicht und damit eine höhere Produktreinheit ermöglicht, die zu einer vereinfachten Aufarbeitung der Hydrierprodukte führt. Gleichzeitig resultiert aus der verbesserten Selektivität des erfindungsgemäß verwendeten Katalysators eine höhere Ausbeute an Amin. Gegenüber den in der Technik üblichen Ausbeuten im Bereich von 96-98 % konnten beim erfindungsgemäßen Verfahren Ausbeuten von 98,5 bis 99,5 % erreicht werden. Durch den geringen Katalysatorgehalt der Hydrierprodukte und den geringeren Gehalt an kann die Aufarbeitung der Hydrierprodukte vereinfacht werden. Auf Grund der geringen Katalysatorkonzentration im Reaktionsgemisch werden die bei den bekannten Verfahren auftretenden Störungen des Verfahrens infolge Katalysatorablagerungen und Verstopfungen im Reaktionssystem vermieden.

Bei dem erfindungsgemäßen Verfahren kann durch das spezielle Trägermaterial überraschenderweise auch die Bildung von feinteiligen Katalysatoranteilen durch Abrieb, mechanische Zerkleinerung usw. weitgehend herabgesetzt werden. Diese Eigenschaft des Katalysators ist besonders bei der Durchführung des Verfahrens in Schleifenreaktoren vorteilhaft, da dort der Katalysator mechanisch stark beansprucht wird. Bei der Abtrennung des Katalysators vom Hydrierprodukt während der Sedimentation wird lediglich katalytisch inaktiver, vergifteter Katalysator in feinteiliger Form und nur in geringen Mengen aus dem Reaktorsystem ausgetragen. Durch Einsatz des speziellen Trägermaterials kann der häufig stattfindende Abbau des Katalysatorträgers, auch als Auslaugung bezeichnet, ebenfalls weitgehend bis vollständig unterdrückt werden. Der entscheidende Vorteil des erfindungsgemäßen Verfahrens ist somit, daß der Katalysatorverbrauch wesentlich gesenkt werden kann. Der Einsatz eines mechanische und chemisch stabilen Katalysators erlaubt es, die Durchmischung im Reaktor sehr intensiv zu gestalten. Dadurch wird ein ungehemmter Stofftransport vom und zum Katalysatorkorn ermöglicht. Der mechanisch und chemisch stabile Katalysator wird in der Suspensionfahrweise wesentlich langsamer zerkleinert als die bei bekannten Verfahren eingesetzten Katalysatoren. Er läßt sich daher gut im Dekantiergefäß sedimentieren und in den Reaktor zurückführen. Die Zerkleinerung der Katalysatorkörner und der damit verbundene Austrag aus dem Reaktorsystem erfolgt erst nach solch einer langen Nutzungsdauer, daß die katalytische Aktivität durch die unvermeidlichen Einflüsse von Katalysatorgiften und organischen Ablagerungsprodukten bereits weitgehend geschädigt wurde. Die Gesamtmenge an ausgetragenem Katalysator sinkt infolge der mechanischen Festigkeit und chemischen Stabilität der Katalysatorteilchen und des dadurch stark verzögerten Abriebs sowie der ausbleibenden Agglomeration hydrieraktiver Spezies deutlich ab. Dadurch werden die Verarbeitungsprobleme des Hydrierprodukts weitgehend beseitigt. Auch die bei den üblichen Verfahren bekannten Störungen durch Zusammenbakken von Katalysatorteilchen im Rückführsystem werden bei dem erfindungsgemäßen Verfahren umgangen.

Die Erfindung und soll in den folgenden Beispielen näher erläutert werden.

### Beispiel 1 (Vergleichsbeispiel)

In einem Laborhydrierreaktor mit einem Volumen von 2.2 l wurden zu einem Gemisch aus 750 g Toluylendiamin (TDA, Isomerengemisch 2,4/2,6 = 80/20) und 750 g Wasser 2.7 bis 4.0 g/min Dinitrotoluol (DNT, Isomerengemisch 2,4/2,6 = 80/20), 2.0 bis 3.0 l/min Wasserstoff und ein Nickelkatalysator mit 55 % Nickelgehalt auf SiO₂ mit einer Nickel-Oberfläche von 45 m²/g und Kristallitgrößen von 8 - 10 nm zugeführt. Die Katalysatorkonzentration im System betrug 4 g/kg Reaktionsvolumen. Die Hydrieraktivität ungefähr 40 bis ungefähr 60 g DNT/g Katalysator * h. Es wurde quasikontinuierlich bei einem Wasserstoffdruck von 25 bar und einer Temperatur von 120°C hydriert. Nach beendeter Reaktion wurde der Katalysator aus dem System abgetrennt und röntgenographisch untersucht. Es war ein deutliches Ni-Kristallitgrößenwachstum auf 16 nm festzustellen. Außerdem wurde die Bildung Ni-haltiger Silikate beobachtet. Das Amin/Wasser-Gemisch wurde anschließend einer Destillation unterworfen. Die Ausbeute an Diamin betrug, bezogen auf eingesetztes Dinitrotoluol, ca. 96 %. Bei der destillativen Aufarbeitung fielen 1.6 % niedrigsiedende Nebenprodukte ("Leichtsieder") und 2.4 % teerartige Produkte ("Hochsieder") an. Der Gehalt an Nitro- bzw. Aminonitroverbindungen im Produktaustrag lag unterhalb der Nachweisgrenze von 10 ppm.

### Beispiel 2 (erfindungsgemäß)

In einem Laborhydrierreaktor mit einem Volumen von 2.2 l wurden zu einem Gemisch aus 750 g Toluylendiamin (TDA, Isomerengemisch 2,4/2,6 = 80/20) und 750 g Wasser 6.0 g/min Dinitrotoluol (DNT, Isomerengemisch 2,4/2,6 = 80/20), 4.4 bis 4.5 l/min Wasserstoff und ein Nickelkatalysator mit 65 % Nickelgehalt auf ZrO₂ mit einer Nickel-Oberfläche von 55 m²/g und Kristallitgrößen von 8 - 9 nm zugeführt. Die Katalysatorkonzentration im System betrug ungefähr 0.5 bis ungefähr 1 g/kg Reaktionsvolumen.. Die Hydrieraktivität ≥ 360 g DNT/g Katalysator * h. Es wurde quasikontinuierlich bei einem Wasserstoffdruck von 25 bar und einer Temperatur von 120°C hydriert. Nach beendeter Reaktion wurde der Katalysator aus dem System abgetrennt und röntgenographisch untersucht. Es war lediglich ein Ni-Kristallitgrößen-Wachstum auf 10 nm festzustellen. Die Bildung nickelhaltiger Verbindungen wurde nicht beobachtet. Das Amin/Wasser-Gemisch wurde anschließend einer Destillation unterworfen. Die Ausbeute an Diamin betrug, bezogen auf eingesetztes Dinitrotoluol, ca. 99 %. Bei der destillativen Aufarbeitung fielen 0.15 % niedrigsiedende Nebenprodukte ("Leichtsieder") und 0.75 % teerartige Produkte ("Hochsieder") an. Der Gehalt an Nitro- bzw. Aminonitroverbindungen im Produktaustrag lag unterhalb der Nachweisgrenze von 10 ppm.

### Beispiel 3 (Vergleichsbeispiel)

In einem Rührkessel von 24 m³ Volumen wurden zu einem Amin/Wasser-Gemisch von 11.2 t TDA, Isomerengemisch 2,4/2,6 = 80/20, und 8.7 t Wasser 2.7 bis 2.81 t/h DNT, Isomerengemisch 2,4/2,6 = 80/20, 2.1 Nm³/h Wasserstoff und 1.0 bis 1.1 kg/h SiO₂-geträgerter Nickelkatalysator mit einem Nickelgehalt von 55 Gew.-%, einer Kristallitgröße von 8 - 10 nm und einer Nickel-Oberfläche von 45 m²/g zugeführt und kontinuierlich bei einem Wasserstoffdruck von 25 bar und einer Temperatur von 115 °C hydriert. Die Zuführung des Wasserstoffs erfolgte im Gegenstrom über die Hohlwelle eines Turbinenrührers. In einem nachgeschalteten Settler wurde der Katalysator abgetrennt und nach Absetzen im Kreislauf wieder dem Reaktor zugeführt. Das Hydrierbad wurde anschließend destillativ aufgearbeitet. Der Katalysatorverbrauch betrug rund 600 g Katalysator/t TDA. Die Ausbeute an Diamin betrug, bezogen auf eingesetztes Dinitrotoluol, ca. 98.4 %. Bei der destillativen Aufarbeitung fielen 0.5 % niedrigsiedende Nebenprodukte ("Leichtsieder") und 1.1 % teerartige Produkte ("Hochsieder") an. Der Gehalt an Nitro- bzw. Aminonitroverbindungen im Produktaustrag lag unterhalb der Nachweisgrenze von 10 ppm.

### Beispiel 4 (erfindungsgemäß)

In einem Rührkessel von 24 m³ Volumen wurden zu einem Amin/Wasser-Gemisch von 11.2 t TDA, Isomerengemisch 2,4/2,6 = 80/20, und 8.7 t Wasser 2.7 bis 2.81 t/h DNT, Isomerengemisch 2,4/2,6 = 80/20, 2.1 Nm³/h Wasserstoff und 0.6 bis 0.65 kg/h ZrO₂-geträgerter Nickelkatalysator mit einem Nickelgehalt von 68 Gew.-%, einer Kristallitgröße von 8 - 10 nm und einer Nickel-Oberfläche von 55 m²/g zugeführt und kontinuierlich bei einem Wasserstoffdruck von 25 bar und einer Temperatur von 115 °C hydriert. Die Zuführung des Wasserstoffs erfolgte im Gegenstrom über die Hohlwelle eines Turbinenrührers. In einem nachgeschalteten Settler wurde der Katalysator abgetrennt und nach Absetzen im Kreislauf wieder dem Reaktor zugeführt. Das Hydrierbad wurde anschließend destillativ aufgearbeitet. Der Katalysatorverbrauch betrug rund 350 g Katalysator/t TDA. Die Ausbeute an Diamin betrug, bezogen auf eingesetztes Dinitrotoluol, ca. 99.2 %. Bei der destillativen Aufarbeitung fielen 0.2 % niedrigsiedende Nebenprodukte ("Leichtsieder") und 0.6 % teerartige Produkte ("Hochsieder") an. Der Gehalt an Nitro- bzw. Aminonitroverbindungen im Produktaustrag lag unterhalb der Nachweisgrenze von 10 ppm.

Die Ausführungsbeispiele machen deutlich, daß das erfindungsgemäße Verfahren eine starke Einsparung an Katalysator und eine Verbesserung der Aminausbeute im Vergleich zu den bekannten Verfahren ermöglicht.

## Patentansprüche

1. Verfahren zur Herstellung von Aminen, wobei mindestens eine Verbindung, die mindestens eine Nitrogruppe aufweist, in Gegenwart eines Trägerkatalysators, der als katalytisch aktives Metall Nickel aufweist, mit Wasserstoff umgesetzt wird, **dadurch gekennzeichnet, daß** der reduzierte und stabilisierte Trägerkatalysator Nickel-Kristallite mit bimodaler Nickel-Kristallitgrößenverteilung mit Maxima bei 30 - 80 Angström und 81 - 150 Angström auf einem Träger aus ZrO₂, ZrO₂₋HfO₂ und/oder SiO₂-ZrO₂ und/oder SiO₂-ZrO₂-HfO₂ enthält und im reduzierten und passivierten Zustand einen Nickelgehalt von 60 - 80 Masseprozent, einen SiO₂-Gehalt von 0 - 20 Masseprozent, einen ZrO₂-Gehalt von 0 - 40 Masseprozent, einen HfO₂-Gehalt von 0 - 4 Masseprozent aufweist, wobei der Gehalt mindestens einer der Verbindungen, aus denen der Träger aufgebaut ist, ungleich 0 Masseprozent beträgt, und nach einer einstündigen Nachreduktion bei 100 °C einen Reduktionsgrad von mindestens 70 % besitzt.

2. Verfahren nach Anspruch 1, **dadurch gekennzeichnet, daß** der Katalysator in einer Menge von 0,1 bis 5 Gew.-%, bezogen auf die Reaktionsmischung, eingesetzt wird.

3. Verfahren nach Anspruch 1, **dadurch gekennzeichnet, daß** der Katalysator in einer Menge von 0,2 bis 2 Gew.-%, bezogen auf die Reaktionsmischung, eingesetzt wird.

4. Verfahren nach Anspruch 1, **dadurch gekennzeichnet, daß** die Umsetzung bei einer Temperatur im Bereich von 80 bis 200°C durchgeführt wird.

5. Verfahren nach Anspruch 1, **dadurch gekennzeichnet, daß** die Umsetzung bei einem Druck im Bereich von 10 bis 40 bar durchgeführt wird.

6. Verfahren nach Anspruch 1, **dadurch gekennzeichnet, daß** als Nitroverbindungen solche mit einer oder mehreren Nitrogruppen und 1 bis 18 Kohlenstoffatomen im Molekül eingesetzt werden.

7. Verfahren nach Anspruch 1, **dadurch gekennzeichnet, daß** als Nitroverbindungen aliphatische Nitroverbindungen, Nitrobenzole, Nitrotoluole, Nitroxylole, Nitronaphthaline, Chlornitrobenzole, Chlornitrotoluole, Nitroaniline, Nitroalkohole sowie beliebige Gemische aus zwei oder mehreren der genannten Nitroverbindungen eingesetzt werden.

8. Verfahren nach Anspruch 1, **dadurch gekennzeichnet, daß** als Nitroverbindungen aromatische Verbindungen mit einer oder mehreren Nitrogruppen eingesetzt werden.

9. Verfahren nach Anspruch 1, **dadurch gekennzeichnet, daß** als Nitroverbindungen Nitrobenzol, Nitrotoluol und/oder Dinitrotoluole eingesetzt werden.

10. Verfahren nach Anspruch 1, **dadurch gekennzeichnet, daß** die Reaktion in einem Rührkessel durchgeführt wird.

11. Verfahren nach Anspruch 1, **dadurch gekennzeichnet, daß** die Umsetzung in einem Schlaufenreaktor durchgeführt wird.

12. Verfahren nach Anspruch 1, **dadurch gekennzeichnet, daß** die Umsetzung in Abwesenheit eines alkoholischen Lösungsmittels durchgeführt wird.

## Claims

1. A process for preparing amines, in which at least one compound containing at least one nitro group is reacted with hydrogen in the presence of a supported catalyst comprising, as catalytically active metal, nickel, wherein the reduced and stabilized supported catalyst comprises nickel crystallites having a bimodal nickel crystallite size distribution having maxima at 30-80 Angström and 81-150 Angström on a support comprising ZrO₂, ZrO₂-HfO₂ and/or SiO₂-ZrO₂ and/or SiO₂-ZrO₂-HfO₂ and in the reduced and passivated state has a nickel content of 60-80 percent by mass, an SiO₂ content of 0-20 percent by mass, a ZrO₂ content of 0-40 percent by mass, an HfO₂ content of 0-4 percent by mass, the content of at least one of the compounds of which the support is composed not being equal to 0 percent by mass, and after further reduction for one hour at 100°C has a degree of reduction of at least 70%.

2. A process as claimed in claim 1, wherein the catalyst is used in an amount of from 0.1 to 5 percent by weight, based on the reaction mixture.

3. A process as claimed in claim 1, wherein the catalyst is used in an amount of from 0.2 to 2 percent by weight, based on the reaction mixture.

4. A process as claimed in claim 1, wherein the reaction is carried out at a temperature in the range from 80 to 200°C.

5. A process as claimed in claim 1, wherein the reaction is carried out at a pressure in the range from 10 to 40 bar.

6. A process as claimed in claim 1, wherein the nitro compounds used have one or more nitro groups and from 1 to 18 carbon atoms in the molecule.

7. A process as claimed in claim 1, wherein the nitro compounds used are aliphatic nitro compounds, nitrobenzenes, nitrotoluenes, nitroxylenes, nitronaphthalenes, chloronitrobenzenes, chloronitrotoluenes, nitroanilines, nitro alcohols or any mixtures of two or more of the abovementioned nitro compounds.

8. A process as claimed in claim 1, wherein the nitro compounds used are aromatic compounds containing one or more nitro groups.

9. A process as claimed in claim 1, wherein the nitro compounds used are nitrobenzene, nitrotoluene and/or dinitrotoluenes.

10. A process as claimed in claim 1, wherein the reaction is carried out in a stirred vessel.

11. A process as claimed in claim 1, wherein the reaction is carried out in a loop reactor.

12. A process as claimed in claim 1, wherein the reaction is carried out in the absence of an alcoholic solvent.

## Revendications

1. Procédé de préparation d'amines, dans lequel au moins un composé qui présente au moins un groupe nitro, est mis à réagir avec de l'hydrogène en présence d'un catalyseur supporté présentant le nickel comme métal à action catalytique, **caractérisé en ce que** le catalyseur supporté réduit et stabilisé contient des cristallites de nickel ayant une distribution bimodale des tailles des cristallites de nickel avec des maxima autour de 30 à 80 angströms et de 81 à 150 angströms sur un support constitué de ZrO₂, ZrO₂-HfO₂, et/ou SiO₂-ZrO₂, et/ou SiO₂-ZrO₂-HfO₂, et à l'état réduit et passivé, présente une teneur en nickel de 60 à 80% en masse, une teneur en SiO₂ de 0 à 20% en masse, une teneur en ZrO₂ de 0 à 40% en masse, une teneur en HfO₂ de 0 à 4% en masse, la teneur d'au moins un des composés, à partir desquels est construit le support, étant différent de 0% en masse, et possède un degré de réduction d'au moins 70% après une post-réduction d'une heure à 100°C.

2. Procédé selon la revendication 1, **caractérisé en ce que** le catalyseur est mis en oeuvre en une quantité de 0,1 à 5% en poids, par rapport au mélange réactionnel.

3. Procédé selon la revendication 1, **caractérisé en ce que** le catalyseur est mis en oeuvre en une quantité de 0,2 à 2% en poids, par rapport au mélange réactionnel.

4. Procédé selon la revendication 1, **caractérisé en ce que** la réaction est réalisée à une température dans la plage de 80 à 200°C.

5. Procédé selon la revendication 1, **caractérisé en ce que** la réaction est réalisée à une pression dans la plage de 10 à 40 bars.

6. Procédé selon la revendication 1, **caractérisé en ce qu'**en tant que composés nitro, ceux ayant un ou plusieurs groupes nitro et 1 à 18 atomes de carbone dans la molécule sont mis en oeuvre.

7. Procédé selon la revendication 1, **caractérisé en ce que** des composés nitro aliphatiques, nitrobenzènes, nitrotoluènes, nitroxylènes, nitronaphtalènes, chloronitrobenzènes, chloronitrotoluènes, nitroanilines, nitroalcools, ainsi que des mélanges quelconques constitués de deux ou plusieurs des composés nitro cités sont mis en oeuvre en tant que composés nitro.

8. Procédé selon la revendication 1, **caractérisé en ce que** des composés aromatiques ayant un ou plusieurs groupes nitro sont mis en oeuvre en tant que composés nitro.

9. Procédé selon la revendication 1, **caractérisé en ce que** le nitrobenzène, le nitrotoluène, et/ou les dinitrotoluènes sont mis en oeuvre en tant que composés nitro.

10. Procédé selon la revendication 1, **caractérisé en ce que** la réaction est réalisée dans un récipient à agitateur.

11. Procédé selon la revendication 1, **caractérisé en ce que** la réaction est réalisée dans un réacteur à écoulement en boucle.

12. Procédé selon la revendication 1, **caractérisé en ce que** la réaction est réalisée en l'absence d'un solvant alcoolique.
